Europäisches Patentamt

European Patent Office

Office européen des brevets

(19)

(11) Veröffentlichungsnummer: **0 074 652**
**B1**

(12) **EUROPÄISCHE PATENTSCHRIFT**

(45) Veröffentlichungstag der Patentschrift:
14.11.84

(51) Int. Cl.³: **C 01 B 33/28**, B 01 J 29/28

(21) Anmeldenummer: 82108413.4

(22) Anmeldetag: 11.09.82

(54) Gallium- und/oder indiumhaltige Zeolithe und Verfahren zu deren Herstellung sowie ihre Verwendung.

(30) Priorität: 16.09.81 DE 3136686

(43) Veröffentlichungstag der Anmeldung:
23.03.83 Patentblatt 83/12

(45) Bekanntmachung des Hinweises auf die Patenterteilung:
14.11.84 Patentblatt 84/46

(84) Benannte Vertragsstaaten:
BE DE FR GB IT NL

(56) Entgegenhaltungen:
WO - A - 80/02026
DE - A - 1 806 154
DE - A - 2 848 849
US - A - 3 431 219
US - A - 4 046 826

(73) Patentinhaber: HOECHST AKTIENGESELLSCHAFT,
Postfach 80 03 20, D-6230 Frankfurt am Main 80 (DE)

(72) Erfinder: Baltes, Herbert, Dr., Johannesallee 24,
D-6230 Frankfurt am Main 80 (DE)
Erfinder: Leupold, Ernst Ingo, Dr., Am Zäunefeld 15,
D-6392 Neu-Anspach (DE)
Erfinder: Litterer, Heinz, Dr., Albert-Schweitzer-Allee 39,
D-6200 Wiesbaden (DE)
Erfinder: Wunder, Friedrich, Dr., Jahnstrasse 46,
D-6093 Flörsheim am Main (DE)

ACTORUM AG

**Beschreibung**

Zeolithe sind kristalline Aluminosilikate, bei denen durch eine dreidimensionale Verknüpfung von $SiO_4$- und $AlO_4$-Tetraedern regelmässige Strukturen mit Hohlräumen und Poren entstehen. Im hydratisierten Zustand sind diese Poren und Hohlräume mit Wasser gefüllt. Dieses lässt sich ohne Beeinflussung der Kristallstruktur entfernen oder durch andere Moleküle ersetzen. Die negativen Ladungen der $AlO_4$-Tetraeder werden durch Kationen kompensiert. Diese können gegen andere positiv geladene Ionen ausgetauscht werden. Die geschilderten Eigenschaften ermöglichen die Verwendung der Zeolithe als Ionenaustauscher, Adsorbentien und Katalysatoren (D.W. Breck, „Zeolite Molecular Sieves", 1974).

Zeolithe des X-, Y-, Mordenit-, Erionit- und Offretit-Typs beispielsweise besitzen als Katalysatoren für Umwandlungsreaktionen von Kohlenwasserstoffen wie Cracken, Hydrocracken oder Isomerisierungen beträchtliches technisches Interesse. Zeolithe vom Pentasil-Typ (z.B. Zeolith ZSM-5) gewinnen als Katalysatoren für die Umwandlung von Methanol zu Kohlenwasserstoffen steigende Bedeutung.

Aufgrund der zahlreichen Einsatzmöglichkeiten als Katalysatoren besteht grosses Interesse an neuen Zeolithen mit spezifischen katalytischen Eigenschaften.

Beispielsweise erhält man sehr interessante Zeolithe, wenn man anstelle von Aluminium und/oder Silicium andere Elemente in das Zeolith-Gerüst einbaut. So wurden unter anderem Zeolithe der Pentasil-Reihe bekannt, die Bor (DE-OS Nr. 2830787), Eisen (DE-OS Nr. 2831611), Arsen (DE-AS Nr. 2830830), Antimon (DE-OS Nr. 2830787), Vanadin (DE-OS Nr. 2831631), Chrom (DE-OS Nr. 2831630), oder Gallium (BE-PS Nr. 882484) auf Tetraederplätzen enthalten.

Gegenstand der Erfindung sind gallium- und/oder indiumhaltige Zeolithe, die dadurch gekennzeichnet sind, dass sie

a) die folgende Zusammensetzung besitzen
$SiO_2 : (0,20 \pm 0,10)[Al_2O_3 + M_2O_3] : (0,15 \pm 0,1)[Na_2O + K_2O] : (0,15 \pm 0,14) R_2O$
ausgedrückt in Molverhältnissen von Oxiden, wobei M gleich Gallium und/oder Indium und R gleich Tetramethylammonium ist, und

b) im Röntgenbeugungsdiagramm die in Tabelle 1 aufgeführten charakteristischen Signale aufweisen

*Tabelle 1*

| Netzebenenabstände d (Å) | relative Intensität $I/I_o$ |
|---|---|
| 11,50 $\pm$ 0,2 | sehr stark |
| 7,56 $\pm$ 0,1 | schwach bis mittel |
| 6,62 $\pm$ 0,1 | mittel bis stark |
| 6,32 $\pm$ 0,1 | schwach |
| 5,74 $\pm$ 0,1 | schwach |
| 4,56 $\pm$ 0,1 | mittel |
| 4,33 $\pm$ 0,1 | mittel bis stark |

| Netzebenenabstände d (Å) | relative Intensität $I/I_o$ |
|---|---|
| 3,76 $\pm$ 0,1 | sehr stark |
| 3,58 $\pm$ 0,1 | mittel bis stark |
| 3,31 $\pm$ 0,1 | schwach |
| 3,15 $\pm$ 0,1 | schwach bis mittel |
| 2,84 $\pm$ 0,1 | stark bis sehr stark |
| 2,68 $\pm$ 0,1 | schwach bis mittel |
| 2,48 $\pm$ 0,1 | schwach |

Hierbei bedeutet $I_o$ die Intensität des stärksten Signals.

Für die Angabe der Intensitäten in Tabelle 1 gilt

| relative Intensität | 100 $I/I_o$ |
|---|---|
| sehr stark | 80 bis 100 |
| stark | 50 bis 80 |
| mittel | 20 bis 50 |
| schwach | 0 bis 20 |

Die erfindungsgemässen Zeolithe besitzen eine dem Offretit (DE-OS Nr. 1806154) ähnliche Struktur, unterscheiden sich jedoch von diesem in der Zusammensetzung.

Für den erfindungsgemässen Zeolith gilt im allgemeinen

$$\frac{Al_2O_3}{Al_2O_3 + M_2O_3} = 0,01 \text{ bis } 0,99$$

vorzugsweise

$$\frac{Al_2O_3}{Al_2O_3 + M_2O_3} = 0,40 \text{ bis } 0,99$$

insbesondere

$$\frac{Al_2O_3}{Al_2O_3 + M_2O_3} = 0,60 \text{ bis } 0,99$$

ausgedrückt in Molverhältnissen der Oxide, wobei M gleich Gallium und/oder Indium ist.

Die erfindungsgemässen Zeolithe lassen sich herstellen, indem man Gallium- und/oder Indiumverbindungen mit Aluminium-, Silicium-, Natrium-, Kalium-, Tetramethylammoniumverbindungen und Wasser mischt und in einem geschlossenen Gefäss erhitzt.

Die Ausgangsverbindungen werden im allgemeinen in folgendem Verhältnis eingesetzt, ausgedrückt in Molverhältnissen der Oxide
$SiO_2 : (0,03 \pm 0,028)Al_2O_3 : (0,03 \pm 0,028)M_2O_3 : (0,2 \pm 0,08)Na_2O : (0,2 \pm 0,15)K_2O : (0,1 \pm 0,08)R_2O : (20 \pm 10)H_2O$
vorzugsweise im Verhältnis
$SiO_2 : (0,03 \pm 0,01)Al_2O_3 : (0,03 \pm 0,028)M_2O_3 : (0,2 \pm 0,08)Na_2O : (0,2 \pm 0,10)K_2O : (0,035 \pm 0,015)R_2O : (20 \pm 10)H_2O$
wobei M gleich Gallium und/oder Indium und R gleich Tetramethylammonium ist.

Als Verbindungen können beispielsweise eingesetzt werden: Kieselsäure, Kaliumsilikat, Natriumsilikat, Aluminiumhydroxid, Aluminiumsulfat, Natriumaluminat, Kaliumaluminat, Aluminiumhalogenide, Aluminiummetahydroxid, Gallium(III)oxid, Gallium(III)nitrat, Gallium(III)sulfat, Gallium(III)halogenide, Gallium(III)hydroxid, Indium(III)oxid, Indium(III)nitrat, Indium(III)sulfat, Indium(III)halogenide, Indium(III)hydroxid, Natriumhydroxid, Natriumsulfat, Natriumhalogenide, Kaliumhydroxid, Kaliumsulfat, Kaliumhalogenide, Tetramethylammoniumhydroxid, Tetramethylammoniumchlorid. Aber auch andere Silicium-, Aluminium-, Gallium-, Indium-, Kalium-, Natrium- und Tetramethylammoniumverbindungen eignen sich für die Herstellung der erfindungsgemässen Zeolithe.

Das Gemisch der jeweils gewählten Verbindungen mit Wasser wird im allgemeinen 12 bis 300 h lang, vorzugsweise 24 bis 200 h lang, auf eine Temperatur zwischen 60 und 150° C, insbesondere zwischen 80 und 140° C, in einem geschlossenen Gefäss erhitzt.

Die gebildeten kristallinen Zeolithe werden in üblicher Weise, z.B. durch Filtration, isoliert, gewaschen und getrocknet. Sie können nach bekannten Methoden in die katalytisch aktiven Formen überführt werden z.B. durch Kalzinierung und/oder Ionenaustausch (D.W. Breck, „Zeolite Molecular Sieves", 1974).

Die erfindungsgemässen Zeolithe zeigen nach ihrer Überführung in die katalytisch aktive Form insbesondere bei der Umwandlung von Methanol zu Kohlenwasserstoffen eine wesentlich geringere Koksabscheidung und deutlich höhere Aktivität als die gallium- bzw. indiumfreien Offretite gemäss DE-OS Nr. 1086154. Diese Reaktion führt man beispielsweise bei Temperaturen von 350 bis 430° C und einem Wasseranteil mit Methanol von 0 bis 80 Gew.-% oder mit Rohmethanol durch.

Die Erfindung soll durch die folgenden Beispiele erläutert werden, wobei die Beispiele aber in keiner Weise einschränkend sein sollen. Alle angegebenen Röntgenbeugungsdaten wurden mit einem computergesteuerten Pulverdiffraktometer D-500 der Firma Siemens aufgenommen. Es wurde Kupfer-K-α-Strahlung verwandt.

*Beispiel 1*

In eine Lösung aus 4,3 g Gallium(III)oxid, 7,2 g Natriumhydroxid, 17,8 g Kaliumhydroxid, 4,5 g Natriumaluminat (54 Gew.-% $Al_2O_3$, 41 Gew.-% $Na_2O$) und 4,2 g Tetramethylammoniumchlorid in 125 g Wasser werden 98 g 40 gew.-%iges kolloidales Kieselgel eingebracht. Die entstandene Mischung wird durch intensives Rühren homogenisiert und 95 h lang auf 95° C in einem geschlossenen Gefäss erhitzt. Das erhaltene Produkt wird abfiltriert, mit Wasser gewaschen und bei 120° C getrocknet.

Die chemische Analyse ergibt folgende Zusammensetzung, ausgedrückt in Molverhältnissen von Oxiden

$SiO_2$:0,088 $Al_2O_3$:0,056 $Ga_2O_3$:0,028
$Na_2O$:0,084 $K_2O$:0,034 $R_2O$
wobei R = Tetramethylammonium ist.

Das Ergebnis der Röntgenbeugungsanalyse ist in Tabelle 2 wiedergegeben.

*Tabelle 2*

| Netzebenenabstände d (Å) | relative Intensität $I/I_o$ |
|---|---|
| 11,48 | 83 |
| 7,52 | 24 |
| 6,60 | 48 |
| 6,34 | 12 |
| 5,73 | 13 |
| 4,58 | 39 |
| 4,34 | 55 |
| 3,75 | 100 |
| 3,58 | 74 |
| 3,31 | 18 |
| 3,15 | 33 |
| 2,86 | 90 |
| 2,67 | 17 |
| 2,48 | 13 |

*Beispiel 2*

Es wird ein Gemisch aus 1,7 g Gallium(III)oxid, 21,4 g Natriumhydroxid, 35,6 g Kaliumhydroxid, 7,0 g Aluminiumhydroxid, 8,4 g Tetramethylammoniumchlorid, 250 g Wasser und 195 g 40 gew.-%igem kolloidalem Kieselgel hergestellt und 72 h lang in einem geschlossenen Gefäss auf 110° C erhitzt. Nach Aufarbeitung wie in Beispiel 1 erhält man ein kristallines Produkt mit folgender Zusammensetzung, ausgedrückt in Molverhältnissen von Oxiden

$SiO_2$:0,101 $Al_2O_3$:0,011 $Ga_2O_3$:0,022
$Na_2O$:0,066 $K_2O$:0,042 $R_2O$
wobei R = Tetramethylammonium ist.

Die Röntgendaten entsprechen den in Tabelle 1 angegebenen.

*Beispiel 3*

Es wird ein Gemisch aus 12,9 g Gallium(III)-oxid, 21,4 g Natriumhydroxid, 35,6 g Kaliumhydroxid, 0,7 g Aluminiumhydroxid, 8,4 g Tetramethylammoniumchlorid, 250 g Wasser und 195 g 40 gew.-%igem kolloidalem Kieselgel hergestellt und 120 h lang in einem geschlossenen Gefäss auf 95° C erhitzt. Nach Aufarbeitung wie in Beispiel 1 erhält man ein kristallines Produkt mit folgender Zusammensetzung, ausgedrückt in Molverhältnissen von Oxiden

$SiO_2$:0,018 $Al_2O_3$:0,102 $Ga_2O_3$:0,031
$Na_2O$:0,076 $K_2O$:0,034 $R_2O$
wobei R = Tetramethylammonium ist.

Die Röntgendaten entsprechen den in Tabelle 1 angegebenen.

*Beispiel 4*

In eine Lösung aus 10,1 g Indiumtrichlorid, 7,2 g Natriumhydroxid, 17,8 g Kaliumhydroxid, 4,5 g Natriumaluminat (54 Gew.-% $Al_2O_3$, 41 Gew.-% $Na_2O$) und 4,2 g Tetramethylammoniumchlorid in 125 g Wasser werden 98 g 40 gew.-%iges kolloidales Kieselgel eingebracht. Die entstandene Mischung wird durch intensives Rühren homogenisiert und 95 h lang auf 95° C in einem geschlossenen Gefäss erhitzt. Das erhaltene Produkt wird abfiltriert, mit Wasser gewaschen und bei 120° C getrocknet. Die chemische Analyse ergibt folgende Zusammensetzung, ausgedrückt in Molverhältnissen von Oxiden

$SiO_2$:0,099 $Al_2O_3$:0,038 $In_2O_3$:0,032
$Na_2O$:0,084 $K_2O$:0,032 $R_2O$

wobei R = Tetramethylammonium ist.

Die Röntgendaten entsprechen den in Tabelle 1 angegebenen.

## Patentansprüche

1. Gallium- und/oder indiumhaltige Zeolithe, dadurch gekennzeichnet, dass sie
a) die folgende Zusammensetzung besitzen
$SiO_2$:$(0,20 \pm 0,10)[Al_2O_3 + M_2O_3]$:$(0,15 \pm 0,1)[Na_2O + K_2O]$:$(0,15 \pm 0,14) R_2O$
ausgedrückt in Molverhältnissen von Oxiden. wobei M gleich Gallium und/oder Indium und R gleich Tetramethylammonium ist, und
b) im Röntgenbeugungsdiagramm die in Tabelle 1 aufgeführten charakteristischen Signale aufweisen

*Tabelle 1*

| Netzebenenabstände d (Å) | relative Intensität $I/I_o$ |
|---|---|
| 11,50 $\pm$ 0,2 | sehr stark |
| 7,56 $\pm$ 0,1 | schwach bis mittel |
| 6,62 $\pm$ 0,1 | mittel bis stark |
| 6,32 $\pm$ 0,1 | schwach |
| 5,74 $\pm$ 0,1 | schwach |
| 4,56 $\pm$ 0,1 | mittel |
| 4,33 $\pm$ 0,1 | mittel bis stark |
| 3,76 $\pm$ 0,1 | sehr stark |
| 3,58 $\pm$ 0,1 | mittel bis stark |
| 3,31 $\pm$ 0,1 | schwach |
| 3,15 $\pm$ 0,1 | schwach bis mittel |
| 2,84 $\pm$ 0,1 | stark bis sehr stark |
| 2,68 $\pm$ 0,1 | schwach bis mittel |
| 2,48 $\pm$ 0,1 | schwach |

wobei $I_o$ die Intensität des stärksten Signals ist.

2. Gallium- und/oder indiumhaltige Zeolithe nach Anspruch 1, dadurch gekennzeichnet, dass gilt

$$\frac{Al_2O_3}{Al_2O_3 + M_2O_3} = 0,01 \text{ bis } 0,99$$

ausgedrückt in Molverhältnissen der Oxide, wobei M gleich Gallium und/oder Indium ist.

3. Gallium- und/oder indiumhaltige Zeolithe nach einem der beiden Ansprüche 1 oder 2, dadurch gekennzeichnet, dass gilt

$$\frac{Al_2O_3}{Al_2O_3 + M_2O_3} = 0,40 \text{ bis } 0,99$$

ausgedrückt in Molverhältnissen der Oxide, wobei M gleich Gallium und/oder Indium ist.

4. Gallium- und/oder indiumhaltige Zeolithe nach einem der Ansprüche 1 bis 3, dadurch gekennzeichnet, dass gilt

$$\frac{Al_2O_3}{Al_2O_3 + M_2O_3} = 0,60 \text{ bis } 0,99$$

ausgedrückt in Molverhältnissen der Oxide, wobei M gleich Gallium und/oder Indium ist.

5. Verfahren zur Herstellung von gallium- und/oder indiumhaltigen Zeolithen nach einem der Ansprüche 1 bis 4, dadurch gekennzeichnet, dass man eine Mischung aus Silicium-, Aluminium-, Kalium- und Tetramethylammoniumverbindungen, Wasser sowie Gallium- und/oder Indiumverbindungen herstellt, die folgende Zusammensetzung hat, ausgedrückt in Molverhältnissen der Oxide

$SiO_2$:$(0,03 \pm 0,028)Al_2O_3$:$(0,03 \pm 0,028)M_2O_3$:$(0,2 \pm 0,08)Na_2O$:$(0,2 \pm 0,15)K_2O$:$(0,1 \pm 0,08)R_2O$:$(20 \pm 10)H_2O$

wobei M gleich Gallium und/oder Indium und R gleich Tetramethylammonium ist, und diese Mischung in einem geschlossenen Gefäss erhitzt.

6. Verfahren nach Anspruch 5, dadurch gekennzeichnet, dass die zu erhitzende Mischung folgende Zusammensetzung hat, ausgedrückt in Molverhältnissen der Oxide

$SiO_2$:$(0,03 \pm 0,01)Al_2O_3$:$(0,03 \pm 0,028)M_2O_3$:$(0,2 \pm 0,08)Na_2O$:$(0,2 \pm 0,10)K_2O$:$(0,035 \pm 0,015)R_2O$:$(20 \pm 10)H_2O$

wobei M gleich Gallium und/oder Indium und R gleich Tetramethylammonium ist.

7. Verwendung von gallium- und/oder indiumhaltigen Zeolithen nach einem der Ansprüche 1 bis 4 als Katalysatoren bei der Herstellung von $C_2$ bis $C_4$-Olefinen aus Methanol.

## Claims

1. Gallium and/or indium-containing zeolites which
a) have the following composition
$SiO_2$:$(0.20 \pm 0.10)[Al_2O_3 + M_2O_3]$:$(0.15 \pm 0.1)[Na_2O + K_2O]$:$(0.15 \pm 0.14) R_2O$
expressed as molar ratio of oxides; M being gallium and/or indium and R being tetramethylammonium, and
b) have the characteristic X-ray diffraction pattern set forth below in Table 1

*Table 1*

| Interplanar Spacing d (Å) | Relative Intensity $I/I_o$ |
|---|---|
| 11.50 $\pm$ 0.2 | very strong |
| 7.56 $\pm$ 0.1 | weak to medium |
| 6.62 $\pm$ 0.1 | medium to strong |
| 6.32 $\pm$ 0.1 | weak |
| 5.74 $\pm$ 0.1 | weak |
| 4.56 $\pm$ 0.1 | medium |
| 4.33 $\pm$ 0.1 | medium to strong |
| 3.76 $\pm$ 0.1 | very strong |
| 3.58 $\pm$ 0.1 | medium to strong |
| 3.31 $\pm$ 0.1 | weak |
| 3.15 $\pm$ 0.1 | weak to medium |
| 2.84 $\pm$ 0.1 | strong to very strong |
| 2.68 $\pm$ 0.1 | weak to medium |
| 2.48 $\pm$ 0.1 | weak |

$I_o$ = intensity of the strongest line or peak.

2. Gallium- and/or indium-containing zeolites as claimed in Claim 1, for which the following is valid

$$\frac{Al_2O_3}{Al_2O_3 + M_2O_3} = 0.01 \text{ to } 0.99$$

expressed as molar ratio of oxides; M being gallium and/or indium.

3. Gallium- and/or indium-containing zeolites as claimed in Claim 1 or 2, for which the following is valid

$$\frac{Al_2O_3}{Al_2O_3 + M_2O_3} = 0.40 \text{ to } 0.99$$

expressed as molar ratio of oxides; M being gallium and/or indium.

4. Gallium- and/or indium-containing zeolites as claimed in claims 1 to 3, for which the following is valid

$$\frac{Al_2O_3}{Al_2O_3 + M_2O_3} = 0.60 \text{ to } 0.99$$

expressed as molar ratio of oxides; M being gallium and/or indium.

5. A process for the manufacture of gallium- and/or indium-containing zeolites as claimed in one of Claims 1 to 4, characterized by preparing a mixture of silicon, aluminium, sodium, potassium and tetramethylammonium compounds, water, and gallium- and/or indium compounds having the following composition, expressed in molar ratio of oxides
$SiO_2:(0.03 \pm 0.028)Al_2O_3:(0.03 \pm 0.028)M_2O_3:(0.2 \pm 0.08)Na_2O:(0.2 \pm 0.15)K_2O:(0.1 \pm 0.08)R_2O:(20 \pm 10)H_2O$
M being gallium and/or indium and R being tetramethylammonium, and heating this mixture in a closed vessel.

6. The process as claimed in Claim 5, wherein the mixture to be heated has the following composition, expressed as molar ratio of oxides
$SiO_2:(0.03 \pm 0.01)Al_2O_3:(0.03 \pm 0.028)M_2O_3:(0.2 \pm 0.08)Na_2O:(0.2 \pm 0.10)K_2O:(0.035 \pm 0.015)R_2O:(20 \pm 10)H_2O$
M being gallium and/or indium, and R being tetramethylammonium.

7. Use of gallium- and/or indium-containing zeolites as claimed in one of Claims 1 to 4 as catalysts for the manufacture of $C_2$ to $C_4$-olefins from methanol.

## Revendications

1. Zéolites contenant du gallium et/ou de l'indium, caractérisées en ce que
a) elles ont la composition suivante
$SiO_2:(0.20 \pm 0.10)[Al_2O_3 + M_2O_3]:(0.15 \pm 0.1)[Na_2O + K_2O]:(0.15 \pm 0.14)R_2O$
exprimée en rapports molaires d'oxydes, le symbole M désignant le gallium et/ou l'indium et le symbole R le radical tétraméthylammonium, et

b) leur diagramme de diffraction des rayons X présente les signaux caractéristiques qui sont indiqués dans le tableau 1

*Tableau 1*

| Distance entre plans réticulaires d (Å) | Intensité relative $I/I_o$ |
|---|---|
| $11,50 \pm 0,2$ | très forte |
| $7,56 \pm 0,1$ | faible à moyenne |
| $6,62 \pm 0,1$ | moyenne à forte |
| $6,32 \pm 0,1$ | faible |
| $5,74 \pm 0,1$ | faible |
| $4,56 \pm 0,1$ | moyenne |
| $4,33 \pm 0,1$ | moyenne à forte |
| $3,76 \pm 0,1$ | très forte |
| $3,58 \pm 0,1$ | moyenne à forte |
| $3,31 \pm 0,1$ | faible |
| $3,15 \pm 0,1$ | faible à moyenne |
| $2,84 \pm 0,1$ | forte à très forte |
| $2,68 \pm 0,1$ | faible à moyenne |
| $2,48 \pm 0,1$ | faible |

$I_o$ désignant l'intensité du signal le plus fort.

2. Zéolites contenant du gallium et/ou de l'indium selon la revendication 1, caractérisées par la relation suivante

$$\frac{Al_2O_3}{Al_2O_3 + M_2O_3} = 0,01 \text{ à } 0,99$$

exprimée en rapports molaires des oxydes, le symbole M désignant le gallium et/ou l'indium.

3. Zéolites contenant du gallium et/ou de l'indium selon l'une des revendications 1 ou 2, caractérisées par la relation

$$\frac{Al_2O_3}{Al_2O_3 + M_2O_3} = 0,40 \text{ à } 0,99$$

exprimée en rapports molaires des oxydes, le symbole M représentant le gallium et/ou l'indium.

4. Zéolites contenant du gallium et/ou de l'indium selon l'une des revendications 1 à 3, caractérisées par la relation

$$\frac{Al_2O_3}{Al_2O_3 + M_2O_3} = 0,60 \text{ à } 0,99$$

exprimée en rapports molaires des oxydes, le symbole M désignant le gallium et/ou l'indium.

5. Procédé de préparation de zéolites contenant du gallium et/ou de l'indium selon l'une des revendications 1 à 4, procédé caractérisé en ce que, à partir de composés du silicium, de l'aluminium, du sodium, du potassium et de tétraméthylammonium, d'eau ainsi que de composés du gallium et/ou de l'indium, on prépare un mélange ayant la composition suivante (exprimée en rapports molaires des oxydes)
$SiO_2:(0,03 \pm 0,028)Al_2O_3:(0,03 \pm 0,028)M_2O_3:(0,2 \pm 0,08)Na_2O:(0,2 \pm 0,15)K_2O:(0,1 \pm 0,08)R_2O:(20 \pm 10)H_2O$
le symbole M désignant le gallium et/ou l'indium et le symbole R représentant le radical tétraméthylammonium, et on chauffe ce mélange dans un récipient fermé.

6. Procédé selon la revendication 5, caractérisé en ce que le mélange à chauffer a la composition suivante (exprimée en rapports molaires des oxydes)

$SiO_2:(0,03 \pm 0,01)Al_2O_3:(0,03 \pm 0,028)M_2O_3:(0,2 \pm 0,08)Na_2O:(0,2 \pm 0,10)K_2O:(0,035 \pm 0,015)R_2O:(20 \pm 10)H_2O$

le symbole M représentant le gallium et/ou l'indium et le symbole R le radical tétraméthylammonium.

7. Application de zéolites contenant du gallium et/ou de l'indium, selon l'une des revendications 1 à 4, comme catalyseurs dans la préparation d'oléfines en $C_2$ à $C_4$ à partir de méthanol.